# EUROPEAN PATENT APPLICATION

(11) **EP 4 814 101 A1**
(43) Date of publication of application: **30.09.2026**
(21) Application number: 25167231.7
(22) Date of filing: 28.03.2025
(51) Int. Cl.: C12M 3/06, B01L 3/00, C12M 1/42

(54) **MICROFLUIDIC DEVICE WITH ELECTRODES**

(71) Applicant: Imec VZW, 3001 Leuven (BE)
(72) Inventor: CHAI, Yoke Chin, 3020 Winksele (BE); CHAN, Boon Teik, 3012 Wilsele (BE)
(74) Representative: Winger

(57) **Abstract**

A method for forming a microfluidic device includes: providing a substrate with electrodes having distal ends buried in the substrate; forming interconnect and dielectric layers; exposing the substrate surface by forming a hole through layers; filling the hole with sacrificial material; forming a composite layer with a first structure as a placeholder for a microfluidic compartment and a second structure enclosing the first structure; selectively etching the substrate to expose electrodes and filled hole surface; and removing sacrificial material, thereby forming a through-hole and a microfluidic compartment fluidically connected. The electrodes form a ring around the hole with the hole positioned within the ring boundary, enabling culturing of three-dimensional biological cell structures.

## Description

### Field of the Invention

The present invention relates to the field of microfluidic devices, and more specifically to microfluidic devices with electrodes e.g., for culturing three-dimensional biological cell structures.

### Background of the Invention

Microfluidic devices have emerged as powerful tools for the study of biological systems, offering precise control over fluid flow and cellular environments at microscale dimensions. These technologies have revolutionized numerous fields, including drug development, disease modeling, and fundamental biological research. Three-dimensional biological cell structures, such as organoids, spheroids, and tissue constructs, represent a significant advancement in modeling human physiology and pathophysiology compared to traditional two-dimensional cell cultures.

The integration of three-dimensional biological structures with microfluidic platforms presents unique technical challenges. Current approaches for cultivating three-dimensional biological structures often struggle to maintain these complex cellular architectures in long-term culture. Vascularization, the development of blood vessel networks within these structures, remains particularly problematic. Without adequate vascularization, three-dimensional cell structures frequently experience limited growth, poor maturation, and eventual necrosis due to insufficient nutrient and oxygen delivery to core regions.

Monitoring cellular activity within three-dimensional structures poses additional challenges. Traditional two-dimensional planar microelectrode arrays (MEAs) effectively capture electrophysiological signals from monolayer cell cultures but provide limited insight into the complex three-dimensional interactions occurring throughout the depth of organoids and other three-dimensional structures. Signals originating from within these structures often remain undetected or poorly characterized using conventional recording approaches.

Precise delivery of compounds to specific regions within three-dimensional biological structures presents further difficulties. Current methods typically expose the entire structure to compounds of interest, lacking the spatial precision that would enable targeted delivery to specific regions or cell populations. This limitation hampers detailed studies of localized responses and region-specific effects.

Genetic manipulation of cells within established three-dimensional structures represents another area where current techniques face limitations. Existing approaches for introducing genetic material or performing genetic reprogramming often require dissociation of the structures or rely on inefficient bulk delivery methods that do not achieve consistent results throughout the structure.

The fabrication of devices capable of addressing these various needs presents significant manufacturing challenges. Creating high aspect ratio structures, integrating fluidic and electronic components, and maintaining biocompatibility while providing necessary functionality remain difficult to achieve simultaneously with current fabrication approaches.

Despite significant advances in technologies for culturing and analyzing three-dimensional biological structures, challenges remain in developing integrated systems that can support various aspects of three-dimensional structure cultivation, monitoring, and manipulation. Addressing some of these challenges could enhance the utility of three-dimensional biological structures in biomedical research and applications.

### Summary of the Invention

It is an object of embodiments of the present invention to advance capabilities for studying and interacting with three-dimensional biological structures. This objective is accomplished by the aspects of the present invention.

In a first aspect, the present invention relates to a method for forming a microfluidic device, comprising i. providing a first substrate and forming within it a first set of one or more electrodes, each electrode having a distal end buried in the substrate, ii. forming a first interconnect layer electrically connecting to each electrode of the first set via a proximal end thereof, iii. forming a dielectric layer over the first interconnect layer, iv. exposing a top surface of the first substrate by forming a hole through at least the dielectric layer, the hole stopping at the substrate surface, v. filling the hole with a sacrificial material, forming a composite layer over the filled hole, said composite layer comprising a first structure laterally overlapping with the filled hole to serve as a temporary placeholder for a microfluidic compartment, and a second structure laterally enclosing the first structure on at least two sides, or entirely enclosing the first structure laterally, vii. selectively etching at least part of the substrate with respect to the first set of electrodes and the filled hole, so as to expose the first set of electrodes, and a surface of the filled hole, and viii. removing the sacrificial material and the first structure, thereby forming a through-hole in the substrate where the filled hole was present and a microfluidic compartment where the first structure was present, said microfluidic compartment being fluidically connected with the through-hole.

In embodiments, the first set of one or more electrodes formed in the first substrate in step i. may form a ring of at least three electrodes, wherein the top surface of the first substrate exposed in step iv. is inside the ring, and wherein the hole is laterally positioned entirely within the boundary of the ring. This configuration allows for effective docking of three-dimensional biological cell structures and promotes vascular in-growth.

In embodiments, the at least three electrodes may be from 3 to 10, more preferably from 4 to 8, yet more preferably from 5 to 7, such as 6 electrodes. From 3 to 10 electrodes provide good structural support and sufficient electrical recording capabilities.

In embodiments, the ring may have an outermost boundary having a maximal length measured parallel to the top surface of the substrate of from 50 to 400 micrometers, preferably from 70 to 300 micrometers, more preferably from 80 to 250 µm. This dimension ensures proper sizing for accommodating various three-dimensional biological cell structure sizes.

In embodiments, the first set of electrodes may be a first set of micro-electrodes. This allows for precise electrical measurements at the micro-scale. Electrical measurement can be measurement of electric signals, e.g., pulse conductive signals from neural or intercell electrical communication; or it can be measurement of bioimpedance, e.g., for evaluating the attachment of a cell to an electrode.

In embodiments, the microfluidic device may be for culturing three-dimensional biological cell structures. This enables advanced biological research applications.

In embodiments, the exposed part of each electrode of the first set obtained after step vii may have a height-on-width ratio of at least two. This high aspect ratio is favorable to adequate docking and allow recording deeper in the three-dimensional biological cell structure while minimizing the risk of damaging it.

In embodiments, step i and step ii may comprise providing the first substrate with a hard mask stack and a lithographic mask, the lithographic mask defining an interconnect design for forming the first interconnect layer, anisotropically etching the hard mask stack by using the lithographic mask, thereby forming first interconnect trenches, providing a new mask over the first interconnect trenches, said new mask defining one or more openings, each opening overlapping with a trench, wherein the openings are for forming the first set of one or more electrodes, anisotropically etching the substrate through the new mask to form a mold for the first set of one or more electrodes, and providing a conductive material in the mold and the trenches, thereby forming the first set of one or more electrodes and the first interconnect layer electrically connecting to each electrode of the first set via a proximal end thereof. This precise fabrication process ensures reliable electrode formation.

In embodiments, the new mask may define a ring of at least three openings, and the substrate is etched anisotropically through the new mask to form a ring-shaped mold for the first set of at least three electrodes. This approach creates a well-defined ring structure.

In embodiments, step iv may comprise providing the dielectric layer with a lithographic mask defining a hole design, and anisotropically etching the dielectric layer by using the lithographic mask, thereby exposing the top surface of the substrate by forming a hole through the dielectric layer, the hole stopping at the substrate surface. This controlled etching process creates precise through-holes.

In embodiments, a ring of at least three electrodes may be formed in step i, wherein the lithographic mask defines a hole design laterally positioned entirely within the boundary of the ring, and wherein the hole formed through the dielectric layer is laterally positioned entirely within the boundary of the ring. This configuration optimizes the spatial relationship between electrodes and through-hole, especially when a three-dimensional biological cell structure is docked by the electrodes.

In embodiments, step vi may comprise providing a first material layer over the filled hole, providing the first material layer with a lithographic mask defining the lateral extent of a temporary placeholder for a microfluidic compartment, anisotropically etching the first material layer by using the lithographic mask, thereby forming the first structure, forming a second material layer over the first structure, and planarizing the second material layer so as to expose a top surface of the first structure and so as to form the second structure. This process facilitates creation of complex microfluidic structures.

In embodiments, the method may further comprise, between step vi and step vii, bonding a second substrate to the composite layer, forming an opening in the second substrate, said opening exposing at least part of the first structure, and filling this opening with a filling material, wherein step viii further comprises removing the filling material after the sacrificial material and the first structure have been removed. This allows for multi-layered device construction.

In embodiments, the second substrate may comprise a semiconductor material, preferably silicon. This enables compatibility with standard semiconductor fabrication processes.

In embodiments, the microfluidic device under construction obtained after step vi may be flipped over before performing step vii. This facilitates access to the backside for further processing.

In embodiments, the method may further comprise, between step iii and iv, forming a second set of one or more electrodes in the substrate, the electrodes of the second set having a different height than the electrodes of the first set, each electrode of the second set having a distal end buried in the substrate, and forming a second interconnect layer electrically connecting to each electrode of the second set via a proximal end thereof, and wherein step vii is also performed selectively with respect to the second set of electrodes, so as to expose at least the distal end of the electrodes of the second set in addition to exposing the first set of one or more electrodes and the surface of the filled hole. This multi-height electrode configuration allows for both centro- and peri-organoid sensing.

In embodiments, the height of the electrodes of the second set may be smaller than the height of the electrodes of the first set. This dimensional difference provides complementary sensing capabilities. Typically, the first set is closer to the through-hole than the second set. A second set comprising smaller electrodes than the first set is typically best suited for peri-organoid sensing while a first set comprising larger electrodes than the second set is typically best suited for centro-organoid sensing.

In embodiments, the electrodes of the second set may be nanoelectrodes. These nanoelectrodes enable intracellular measurements at the nanoscale.

In embodiments, the method may further comprise, between steps v and vi, forming a third set of one or more electrodes in the sacrificial material and in the first substrate, each electrode of said third set having a distal end buried in the substrate, having a proximal end laterally surrounded by the sacrificial material, and forming a third interconnect layer electrically connecting each electrode of the third set via their proximal end. This third set of one or more electrodes, also herein called center electrodes, enables additional functionality such as electroporation.

In embodiments, the third set may consist of a single electrode. This central electrode provides a focused electrical field.

In embodiments, at least one electrode among the first, second, and third set may be formed to be hollow, the method comprising forming said at least one electrode with a conductive outer shell and a sacrificial inner core, forming a corresponding interconnect structure that comprises a conductive outer shell and a sacrificial inner core, wherein the conductive outer shell of the at least one electrode is electrically connected to the conductive outer shell of the at least one interconnect structure via a proximal end of said at least one electrode, and wherein the inner core of said at least one electrode is in direct physical contact with the inner core of said interconnect structure via a proximal end of said at least one electrode, selectively removing a distal portion of the conductive outer shell of said at least one electrode, and subsequently removing the sacrificial inner cores of said at least one electrode and the corresponding interconnect structure, thereby resulting in a hollow electrode with an open distal end that is fluidically connected to a hollow interconnect structure at its proximal end. This hollow electrode configuration enables molecular delivery capabilities.

In embodiments, steps (a) and (b) may comprise performing step (i) so that at least one electrode of the first set is formed and comprises: a conductive outer shell forming the outer surface of said at least one electrode, and a sacrificial inner core, wherein the interconnect layer comprises at least one interconnect structure comprising: a conductive outer shell, and a sacrificial inner core, wherein the conductive outer shell of the at least one electrode is electrically connected to the conductive outer shell of the at least one interconnect structure via a proximal end of said at least one electrode, wherein the inner core of said at least one electrode is in direct physical contact with the inner core of said interconnect structure via a proximal end of said at least one electrode, wherein step (c) comprises, during step vii, removing a distal portion of the conductive outer shell of said at least one electrode, and wherein step (d) comprises, after step vii, removing the sacrificial inner core from both said at least one electrode and the interconnect structure, thereby forming said at least one electrode of the first set, wherein said at least one electrode of the first set is hollow with an opening at its distal end, and converting the interconnect structure into a hollow interconnect structure, said hollow electrode being in direct fluidic communication with said hollow interconnect structure. This detailed fabrication approach ensures hollow electrodes with intact electrical functionality while enabling fluidic transport.

In embodiments, steps (a) and (b) may comprise forming the third set of at least one electrode between steps v and vi so that the at least one electrode of the third set comprises: a conductive outer shell forming the outer surface of the central electrode, and a sacrificial inner core, wherein the third interconnect layer comprises an interconnect structure, said interconnect structure comprising: a conductive outer shell, and a sacrificial inner core, wherein the conductive outer shell of said at least one electrode of the third set is electrically connected to the conductive outer shell of the interconnect structure via a proximal end of said at least one of the third set electrode, wherein the inner core of said at least one electrode of the third set is in direct physical contact with the inner core of the interconnect structure via a proximal end of said at least one electrode of the third set, and wherein step (c) comprises, during step vii, removing a distal portion of the conductive outer shell of said at least one electrode of the third set, and wherein step (d) comprises, after step vii, removing the sacrificial inner core from both said at least one electrode of the third set and the interconnect structure, thereby forming said at least one electrode of the third set, wherein said at least one electrode of the first set is hollow with an opening at its distal end, and converting the interconnect structure into a hollow interconnect structure, said hollow electrode being in direct fluidic communication with said hollow interconnect structure. This configuration enables fluid delivery through a central electrode for targeted manipulation of organoids.

In embodiments, the first substrate may comprise a semiconductor material, preferably silicon. This material choice provides excellent compatibility with established microelectronic fabrication techniques and enables high-precision features.

In embodiments, the electrodes of the first set may be spaced apart, preferably regularly spaced. This regular spacing ensures uniform coverage around organoids for consistent measurements.

In embodiments, the largest distance between any two neighboring electrodes of the first set may be from 100 to 200 µm. This spacing optimizes electrode density for effective organoid interaction while maintaining sufficient separation for individual signal discrimination.

In embodiments, the exposed part of each electrode of the first set obtained after step vii may have a height-to-width ratio of from 2 to 70, preferably from 3 to 50, more preferably from 5 to 30. This aspect ratio range balances mechanical stability with the ability to reach into three-dimensional tissue structures.

In embodiments, step iv may be performed so that, after step viii, the first set of one or more electrodes and the through-hole are arranged so that the first set of one or more electrodes is suitable for docking a three-dimensional biological cell structure so that the three-dimensional biological cell structure covers at least partly the through-hole. This arrangement facilitates proper organoid positioning for vascularization through the open port.

In embodiments, step iv may be performed so that, after step viii, the first set of one or more electrodes and the through-hole are arranged so that the first set of one or more electrodes is at all points laterally separated from a top surface of the through-hole by a distance of at most 10%, preferably at most 5%, yet more preferably at most 2% of the largest lateral dimension of the top surface of the through-hole. This precise spacing ensures optimal proximity between electrodes and the through-hole for effective organoid docking.

In embodiments, step iv may be performed so that, after step viii, the first set of one or more electrodes and the through-hole are arranged so that all electrodes of the first set are at a same distance from the through-hole. This uniform distance creates consistent conditions for organoid interaction across all electrodes.

In embodiments, the three-dimensional biological cell structure may be a type of three-dimensional biological cell structure that requires vascularization to survive. This application leverages the through-hole design to provide essential vascular access to sustain complex tissue structures.

In embodiments, the three-dimensional biological cell structure may be selected from organoids, spheroids, and three-dimensional tissue engineering constructs. These specific tissue types benefit from the device's architecture for proper development and function.

In embodiments, in step i, the first set of one or more electrodes may be formed in the substrate so that their longitudinal axis is perpendicular to a top surface of the substrate. This orientation maximizes electrode contact with three-dimensional tissue structures.

Any feature of any embodiment of the first aspect may be as correspondingly described in the second or third aspect.

In a second aspect, the present invention relates to a microfluidic device, comprising a microfluidic compartment, an electrode compartment comprising a first set of one or more electrodes extending from a proximal end closest to the microfluidic compartment to a distal end farthest from the microfluidic compartment, a separation layer between the microfluidic compartment and the electrode compartment, the separation layer comprising a first interconnect layer electrically connected to each electrode of the first set at the proximal end thereof; a dielectric layer between the first interconnect layer and the microfluidic compartment, and a hole extending through the separation layer, thereby ensuring fluid communication between the electrode compartment and the microfluidic compartment.

In embodiments, each electrode of the first set may have an exposed height-to-width ratio of at least two. This high aspect ratio provides improved electrical performance.

In embodiments, the microfluidic device and the electrode compartment may be for culturing three-dimensional biological cell structures. This configuration supports complex biological research applications.

In embodiments, the first set of one or more electrodes may be a ring of at least three electrodes and the hole may be laterally positioned entirely within the boundary of the ring. This arrangement creates an effective docking station for three-dimensional biological cell structures with central access for vascularization.

In embodiments, the at least three electrodes of the first set may be from 3 to 10, more preferably from 4 to 8, yet more preferably from 5 to 7, such as 6. From 3 to 10 electrodes provides good structural support and sufficient electrical recording capabilities. Electrical measurement can for instance be measurement of electric signals or it can be the measurement of bioimpedance.

In embodiments, the ring may have an outermost boundary having a maximal length measured parallel to the top surface of the substrate of from 50 to 400 micrometers, preferably from 70 to 300 micrometers, more preferably from 80 to 250 µm. This sizing accommodates various organoid dimensions.

In embodiments, the first set of one or more electrodes may be a first set of one or more micro-electrodes. This allows for precise electrical measurements at the micro scale.

In embodiments, the microfluidic device may further comprise a second set of one or more electrodes extending from a proximal end closest to the microfluidic compartment to a distal end farthest from the microfluidic compartment, the electrodes of the second set having a different height than the electrodes of the first set, and a second interconnect layer in the separation layer, electrically connecting to the electrodes of the second set via a proximal end thereof. This dual-height configuration enables complementary sensing capabilities.

In embodiments, at least one electrode among the first, second, and third set may be hollow and comprises a conductive outer shell forming the outer surface of the electrode, wherein the interconnect structure is hollow and has a conductive outer shell, and wherein a proximal end of said hollow electrode is fluidically connected to the hollow interconnect structure. This hollow structure enables molecular delivery.

In embodiments, each of the electrodes of the first set may be hollow and comprise a conductive outer shell forming the outer surface of the electrode, and the first interconnect layer comprises a hollow interconnect structure, the hollow interconnect structure having a conductive outer shell and being fluidically connected to the hollow micro-electrode at the proximal end thereof. This fully hollowed electrode ring enables circumferential fluid delivery around three-dimensional biological cell structures.

In embodiments, the electrode compartment may further comprise: a third set of one or more electrodes, each electrode of the third set: extending from a proximal end closest to the microfluidic compartment to a distal end farthest from the microfluidic compartment, being laterally surrounded by the first set of one or more electrodes if the first set comprises a plurality of electrodes, being in the through-hole while being laterally spaced apart from the inner walls of the through-hole, thereby ensuring fluidic connection between the microfluidic compartment and the electrode compartment, and a third interconnect layer in the separation layer, electrically connecting to the electrodes of the third set via a proximal end thereof. This central electrode configuration enables electroporation or central monitoring while maintaining fluid flow through the through-hole.

In embodiments, each electrode of the third set may be hollow, comprise a conductive outer shell forming the outer surface of the electrode except at a distal open end thereof, wherein the third interconnect layer comprises a hollow interconnect structure comprising a conductive outer shell, and wherein the conductive outer shell of each hollow electrode of the third set is electrically connected to the conductive outer shell of the hollow interconnect structure via a proximal end of the hollow electrode of the third set, wherein the hollow electrode of the third set is fluidically connected to a hollow interconnect structure. This hollow central electrode(s) enables targeted fluid delivery through the center of the device while maintaining electrical functionality.

In embodiments, the at least three electrodes of the first set may be spaced apart, preferably regularly spaced. This regular spacing ensures uniform interaction with organoids for consistent monitoring.

In embodiments, the largest distance between any two neighboring electrodes of the first set may be from 100 to 200 µm. This spacing optimizes electrode density for effective electrical monitoring while maintaining fabrication feasibility.

In embodiments, the exposed part of each electrode of the first set obtained after step vii may have a height-to-width ratio of from 2 to 70, preferably from 3 to 50, more preferably from 5 to 30. This aspect ratio range ensures mechanical stability while enabling effective penetration into three-dimensional tissue structures.

In embodiments, the microfluidic device may comprise the three-dimensional biological cell structure docked by the one or more electrodes of the first set. This configuration demonstrates the functional application of the device for supporting organoid culture.

In embodiments, the three-dimensional biological cell structure may be a type of three-dimensional biological cell structure that requires vascularization to survive. This application leverages the through-hole design to provide essential vascular access to complex tissue structures.

In embodiments, the three-dimensional biological cell structure may be selected from organoids, spheroids, and three-dimensional tissue engineering constructs. These tissue types benefit from the specialized device architecture for proper development and function.

In embodiments, the one or more electrodes of the first set may be at all points laterally separated from a top surface of the through-hole by a distance of at most 10%, preferably at most 5%, yet more preferably at most 2% of the largest lateral dimension of the top surface of the through-hole. This precise spacing ensures optimal proximity for tissue interaction while maintaining fluid access.

In embodiments, all electrodes of the first set may be at a same distance from the through-hole. This uniform spacing creates consistent conditions for tissue interaction across all electrodes.

In embodiments, the electrodes of the first set may have their longitudinal axis perpendicular to a top surface of the dielectric layer. This orientation maximizes the effectiveness of electrode-tissue interactions.

Any feature of any embodiment of the second aspect may be as correspondingly described in the first or third aspect.

In a third aspect, the present invention relates to a system for culturing and monitoring three-dimensional biological cell structures, comprising the microfluidic device according to any embodiments of the second aspect; a fluid control unit configured to control fluid flow through the microfluidic compartment and the through-hole; and an electronic measurement unit configured to receive and process electrical signals from at least the first set of one or more electrodes and from the second and/or third set if present.

Any feature of any embodiment of the third aspect may be as correspondingly described in the first or second aspect.

It is an advantage of embodiments of the present invention that a bio-docking station can include a through-hole for fluid communication with an underlying vascular bed.

It is an advantage of embodiments of methods of the present invention that they permit the formation of high aspect-ratio microelectrodes, thereby allowing three-dimensional docking of biological structures.

It is an advantage of embodiments of the present invention that electrodes of the second set, e.g., nanoelectrodes, can be arranged to record peripheral (e.g., peri-organoid) intracellular signals in the three-dimensional biological cell structure.

It is an advantage of embodiments of the present invention that hollow electrodes, e.g., hollow electrodes of the first set, can enable targeted delivery of molecules of interest in the three-dimensional biological cell structure.

It is an advantage of embodiments of the present invention that hollow electrodes, e.g., electrodes of the second set, can be used for on-chip electroporation of genetic materials.

It is an advantage of embodiments of the present invention that separate electrode sets with different heights can provide biosensing capabilities at the core and the periphery of three-dimensional biological cellular material (e.g., intra- and peri-organoid biosensing capabilities).

It is an advantage of embodiments of the present invention that open ports can ensure fluidic connection between an electrode compartment and a microfluidic compartment.

It is an advantage of embodiments of the present invention that a microfluidic cavity can be integrated to allow controlled perfusion and nutrient delivery.

It is an advantage of embodiments of the present invention that silicon-based fabrication processes can be used to create complex electrode and fluidic architectures in a single chip.

It is an advantage of embodiments of the present invention that organoid viability and growth can be supported by vascularization enabled through the through-hole.

It is an advantage of embodiments of the present invention that real-time monitoring of organoid electrophysiological signals can be achieved with embedded electrode structures.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig.1 is a CAD drawing of a microfluidic device (center pane) showing a bottom part comprising micro- and nano-electrode arrays surrounding an open port, and a top part comprising concave receivers to confine three-dimensional cell structures according to embodiments of the present invention. A close-up (left pane) and an exploded view (right pane) are also depicted.
Fig.2 is a schematic view of interconnect designs for the microfluidic device according to embodiments of the present invention.
Fig.3 is a schematic top view showing five different configurations of hollow and non-hollow electrode arrays with an open port according to embodiments of the present invention.
Fig.4 is a schematic top view of a first design configuration of the microfluidic device featuring a bio-dock and open port for organoid docking according to embodiments of the present invention.
Fig.5 is a schematic top view of a second design configuration of the microfluidic device featuring 3D hollow electrodes of the first set at the bio-dock according to embodiments of the present invention.
Fig.6 is a schematic top view of a third design configuration (option 1) of the microfluidic device featuring an electrode of the third set at the center of the bio-dock according to embodiments of the present invention.
Fig.7 is a schematic top view of a third design configuration (option 2) of the microfluidic device featuring a hollow electrode of the third set at the center of the bio-dock surrounded by non-hollow electrodes of the first set according to embodiments of the present invention.
Fig.8 is a schematic top view of a third design configuration (option 3) of the microfluidic device featuring a hollow electrode of the third set at the center of the bio-dock surrounded by hollow electrodes of the first set according to embodiments of the present invention.
Fig.9 is a process flow diagram illustrating the method for forming the first design configuration of the microfluidic device according to embodiments of the present invention.
Fig.10 is a process flow diagram illustrating the method for forming the second design configuration of the microfluidic device according to embodiments of the present invention.
Fig.11 is a process flow diagram illustrating the method for forming the third design configuration (option 1) of the microfluidic device according to embodiments of the present invention.
Fig.12 is a process flow diagram illustrating the method for forming the third design configuration (option 2) of the microfluidic device according to embodiments of the present invention.
Fig.13 is a process flow diagram illustrating the method for forming the third design configuration (option 3) of the microfluidic device according to embodiments of the present invention.
Fig. 14 is a flowchart of embodiments of the first aspect of the present invention.
Fig. 15 is a diagram of an embodiment of the third aspect of the present invention.

In the different figures, the same reference signs refer to the same or analogous elements.

### Detailed description of Illustrative Embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The following terms are provided solely to aid in the understanding of the invention.

As used herein, and unless otherwise specified, the term "microfluidic device" refers to a structure or assembly designed to manipulate small volumes of fluids in microscale channels, compartments, or through-holes.

As used herein, and unless otherwise specified, the term "first substrate" refers to a base material, e.g., comprising a semiconductor (such as silicon), on or within which electrodes, interconnect layers, dielectric layers, and holes are formed. Examples of specific embodiments of a "first substrate" include a silicon wafer, a glass wafer, or any combination of materials with sufficient structural integrity to support formation of stacked layers and etched features.

As used herein, and unless otherwise specified, the phrase "distal portion" or "distal end" of an electrode refers to the end or portion of the electrode that is farthest from the interconnect layer or from any external electrical contact point. Examples of specific embodiments of a "distal end" include the tip of a tall pillar electrode embedded in a substrate, the apex of a metal spike formed by anisotropic etching, or the open tip of a hollow electrode.

As used herein, and unless otherwise specified, the phrase "buried in the substrate" refers to a configuration in which at least a portion of an electrode (such as its distal end) or other structure is embedded below the top surface of the substrate, such that the embedded portion is covered by substrate material. Examples of specific embodiments of a "buried" feature include deep-etched electrodes whose lower ends reside within the bulk of a silicon wafer and conductive structures or cavities fully encased by the substrate material.

As used herein, and unless otherwise specified, the term "interconnect layer" refers to a layer or set of conductive traces that electrically connect electrodes to external circuitry or other device components. Examples of specific embodiments of an "interconnect layer" include thin-film metal lines (such as aluminum, gold, or copper), multilayer metallization stacks separated by dielectric layers, and any patterned metallization (e.g., patterned using etching or lift-off processes).

As used herein, and unless otherwise specified, the term "dielectric layer" refers to an electrically insulating layer. It may for instance be formed over or under an interconnect layer, typically for the purpose of electrical isolation, passivation, or protection. Examples of specific embodiments of a "dielectric layer" include silicon oxide, silicon nitride, polymeric dielectrics, or multi-layer stacks of inorganic and/or organic thin films.

As used herein, and unless otherwise specified, the term "forming a hole" refers to producing an opening that extends through at least one layer (for example the dielectric layer and interconnect layer) down to a specified stop layer or the top surface of the underlying substrate. Examples of specific embodiments of forming such a hole include reactive ion etching (RIE) through a dielectric layer, laser drilling, or chemical etching processes that reach the desired depth and expose the top surface of the substrate.

As used herein, and unless otherwise specified, the term "sacrificial material" refers to any material deliberately deposited to occupy a space temporarily and then removed in a subsequent process step. Examples of specific embodiments of a "sacrificial material" include photoresist, polymeric resins, waxes, silicon oxide, or other etchable materials that can be selectively removed without damaging the surrounding layers or structures.

As used herein, and unless otherwise specified, the term "composite layer" refers to a combined structure formed of at least two materials or sub-layers, such as a first structure (e.g., a placeholder or patterned layer) and a second structure (e.g., a planarizing layer), that together form a functional layer over the substrate. An example of a specific embodiments of a "composite layer" include a patterned (e.g., amorphous silicon) layer surrounded by a second layer (e.g., a silicon nitride layer) laterally enclosing the patterned layer.

As used herein, and unless otherwise specified, the expression "filling the hole with a sacrificial material" refers to the act of depositing or introducing a removable material into the hole so that it occupies substantially all of the hole's volume, creating a temporary fill.

As used herein, and unless otherwise specified, the phrase "temporary placeholder for a microfluidic compartment" refers to a structure formed in a region where a microfluidic compartment is intended to exist, so that the placeholder is subsequently removed to create the final compartment. Examples of specific embodiments of a "temporary placeholder" include photo-patterned polymer layers, sacrificial photoresists, or other removable layers (e.g., an amorphous silicon layer) that define the future compartment's boundaries.

As used herein, and unless otherwise specified, the term "microfluidic compartment" refers to a defined volume or chamber within the microfluidic device through which fluid can flow or be contained, and which can be used to house cells, reagents, or other materials. Examples of specific embodiments of a "microfluidic compartment" include a culture well for biological cells, a fluid mixing chamber in a lab-on-chip device, a perfusion channel through which growth media is circulated, or a vascular bed in which vascularization of a three-dimensional cell structure takes place.

As used herein, and unless otherwise specified, the phrase "removing the sacrificial material" or "removing the sacrificial cores" refers to subjecting the device to a selective etch, dissolution, or ashing step that eliminates the temporary or core material without substantially affecting the permanent device structures. Examples of specific embodiments of removing sacrificial materials include oxygen plasma ashing of photoresist, wet solvent dissolution of polymeric fillers, or selective isotropic etching of sacrificial silicon-based compounds such as silicon oxide or amorphous silicon.

As used herein, and unless otherwise specified, the term "through-hole" refers to a hole or opening that passes completely through one or more layers, allowing fluid, gas, or other matter to pass from one side to the other. Examples of specific embodiments of a "through-hole" include a fluid channel etched through a substrate linking the front side microfluidic chamber to electrodes on the backside, or an opening that reaches a second substrate and facilitates external fluidic connections.

As used herein, and unless otherwise specified, the expression "fluidically connected" refers to a condition in which two or more spaces or compartments share a continuous fluid pathway, thereby allowing the flow or exchange of fluid between them. Examples of specific embodiments of "fluidic connection" include a through-hole that connects a microfluidic compartment to an electrode compartment, or a channel etched in the substrate that interlinks separate chambers.

As used herein, and unless otherwise specified, the term "ring" of electrodes refers to an arrangement of three or more electrodes disposed along a virtual ring, thereby forming a perimeter. Examples of specific embodiments of a "ring" of electrodes include a circular array of six electrodes, a hexagonal pattern of electrodes, or a rectangular arrangement of four electrodes.

As used herein, and unless otherwise specified, the term "three-dimensional biological cell structure" refers to any cell aggregate or engineered tissue that extends in three dimensions. It typically has structural and/or functional properties that differ from a simple monolayer of cells. Examples of specific embodiments of a "three-dimensional biological cell structure" include organoids derived from stem cells, spheroids formed from tumor cells, or artificially engineered tissue microconstructs requiring perfusion.

As used herein, and unless otherwise specified, the term "height-on-width ratio" or "height-to-width ratio" refers to the ratio of an exposed part of an electrode's longitudinal dimension (height) extending from its distal end to its proximal end (or from the substrate surface to the distal end) to its lateral dimension (width) at measured at the base of the exposed part of the electrode. Examples of specific embodiments of "height-to-width ratio" include an electrode that is 100 µm tall and 20 µm wide, giving a ratio of 5, or electrodes that are 300 µm tall and 30 µm wide, giving a ratio of 10.

As used herein, and unless otherwise specified, the expression "anisotropically etching" refers to an etching process in which material is removed at different rates in different directions, typically resulting in highly directional or straight-walled features. Examples of specific embodiments of anisotropic etching include deep reactive ion etching (DRIE) of silicon, plasma etching of dielectric layers using directional ion bombardment, or crystallographically selective wet-etching of a substrate.

As used herein, and unless otherwise specified, the phrase "bonding a second substrate" refers to attaching another substrate (which may also be a semiconductor wafer or any other suitable material) to the composite layer. This encompasses all possible means of attaching that second substrate to the composite layer of the device under construction, typically using adhesive layers, thermal fusion bonding, anodic bonding, or other bonding techniques. Examples of specific embodiments include wafer-to-wafer alignment and bonding in a cleanroom environment, adhesive bonding with polymer adhesives, or hermetic glass-silicon anodic bonding.

As used herein, and unless otherwise specified, the term "flipped over" refers to physically inverting the orientation of the partially fabricated microfluidic device such that processing or subsequent etching can occur from the opposite side. Examples of specific embodiments of "flipping over" include inverting a wafer so that backside etching can reveal distal ends of electrodes, or orienting a device so that lithographic alignment is performed from the top side after a step on the bottom side.

As used herein, and unless otherwise specified, the term "micro-electrodes" refers to electrodes having at least one critical dimension in the micrometer range, typically from 1 µm up to 200 µm in width. An example of a specific embodiment of "micro-electrodes" include micro-pillars for electrophysiological measurements.

As used herein, and unless otherwise specified, the term "nanoelectrodes" refers to electrodes characterized by at least one critical dimension (such as diameter or width) on the nanometer scale, typically below 1 µm such as from 200 nm to 900 nm. This can for instance be the case for the electrodes of the second set. Examples of specific embodiments of "nanoelectrodes" include metallic structures such as metal pillars or posts with sub-micron diameters. They enable high spatial resolution for sensing or stimulation at the nanoscale.

As used herein, and unless otherwise specified, the term "outer shell" when referring to a hollow electrode (or a hollow interconnect) refers to the conductive material forming the perimeter or walls of the electrode or interconnect structure. In embodiments, an interior volume (inner core) can be removed to render the electrode or interconnect hollow.

As used herein, and unless otherwise specified, the term "inner core" when referring to a hollow electrode (or a hollow interconnect) refers to a removable material occupying the interior volume of the electrode or interconnect structure that is subsequently sacrificed or etched out to produce a hollow region.

As used herein, and unless otherwise specified, the expression "largest distance between neighboring electrodes" refers to the maximum lateral spacing measured between any two adjacent electrodes in a set. Examples of specific embodiments include distances of 100 µm, 150 µm, or 200 µm, depending on the desired density of electrodes around a central hole or microfluidic compartment.

As used herein, and unless otherwise specified, the phrase "dock a three-dimensional biological cell structure" or "suitable for docking a three-dimensional biological cell structure" refers to the arrangement of electrodes and through-holes so that a 3D cell construct can be prevented from displacing by the electrodes while still having access to fluid flow through the hole. An examples of a specific embodiment includes a ring of micro-electrodes cradling an organoid around a central opening for perfusion.

As used herein, and unless otherwise specified, the phrase "largest lateral dimension of the top surface of the through-hole" refers to the maximum cross-sectional width or diameter of the opening at the topmost plane where the through-hole is accessed. In other words, it is the longest straight-line distance across the opening of the through-hole, measured in the plane at the uppermost boundary where the through-hole begins, regardless of the shape of the opening. Examples of specific embodiments include a circular through-hole with a diameter of 100 µm (largest lateral dimension of the top surface is 100 µm), a rectangular through-hole with a 300 µm × 200 µm opening (largest lateral dimension of the top surface is the 361 µm diagonal), or an oval-shaped hole measured at its longest axis.

As used herein, and unless otherwise specified, the term "vascularization" refers to the presence or formation of blood vessel structures within or around a three-dimensional biological cell structure, enabling perfusion of nutrients and waste removal. Examples of specific embodiments of "vascularization" include vessel network formation within organoids, or artificially induced vessel growth by co-culturing endothelial cells with other cell types.

As used herein, and unless otherwise specified, the term "same distance from the through-hole" in the context of electrodes refers to an arrangement in which all electrodes are equidistant laterally from the perimeter of a through-hole. Examples of specific embodiments include a circular array of six electrodes uniformly spaced around a center hole or a rectangular ring of electrodes equally offset from a through-hole's boundary.

As used herein, and unless otherwise specified, the term "organoids" refers to three-dimensional cell structures grown from stem cells or organ-specific progenitor cells that self-organize and mimic the architecture and function of native organs. They typically do so to a significant extent. Examples of specific embodiments of organoids include brain organoids, gut organoids, liver organoids, and kidney organoids used in disease modeling and drug screening.

As used herein, and unless otherwise specified, the term "electrode compartment" refers to the volume or region containing one or more electrodes.

As used herein, and unless otherwise specified, the term "separation layer" refers to a membrane, layer, or layer stack arranged between a microfluidic compartment and an electrode compartment, which may comprise interconnect layers and dielectric layers. It may be crossed by a hole to enable fluid communication.

As used herein, and unless otherwise specified, the term "electronic measurement unit" refers to any apparatus or circuitry configured to monitor, record, or process electrical signals received from one or more electrodes within the microfluidic device. Examples of specific embodiments of an "electronic measurement unit" include potentiostats for electrochemical sensing, impedance analyzers for cell viability measurements, and multichannel recording systems for electrophysiological data acquisition.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

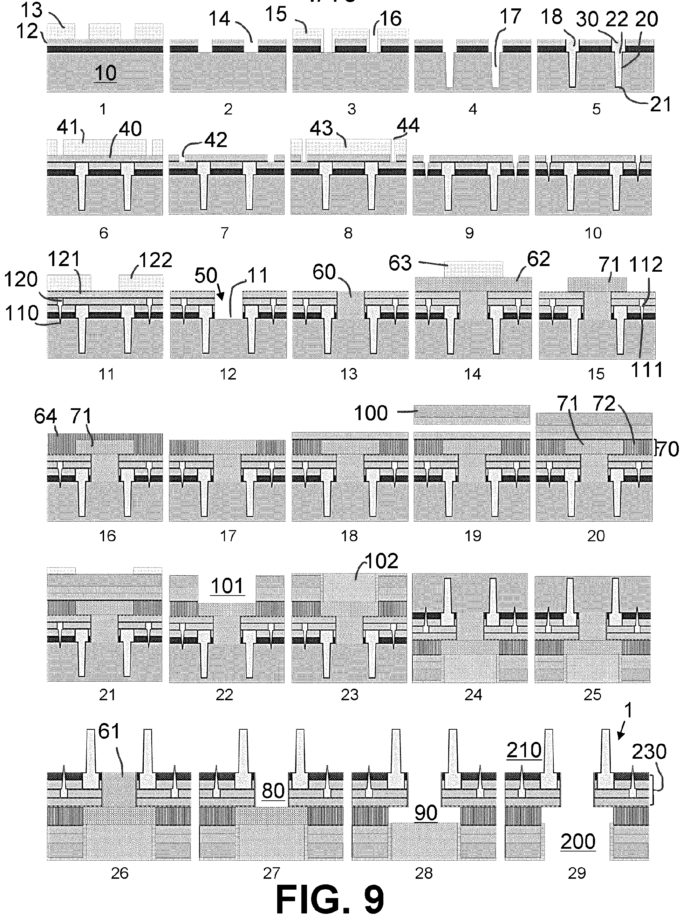

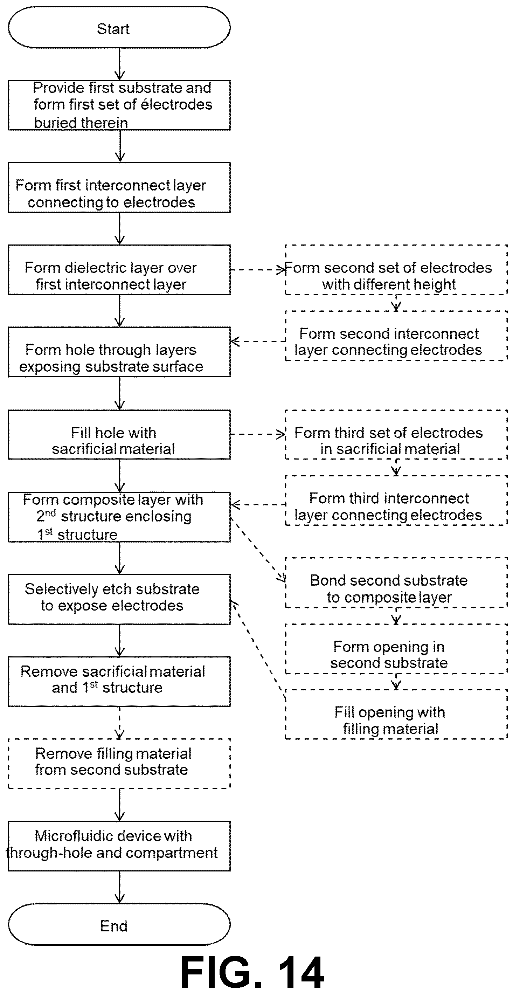

In a first aspect, the present invention relates to a method for forming a microfluidic device (1). A flow chart of the method is depicted in Figure 14. Example embodiments of the method are depicted in Figures 9 to 13 for the case where both a first set and a second set of electrodes are formed. In its most general form, depicted in Figure 14, the method comprises forming a first set of electrodes and only optionally a second and/or a third set. The method comprises i. providing a first substrate (10) and forming within it a first set of one or more electrodes (20), each electrode having a distal end (21) buried in the substrate (10), ii. forming a first interconnect layer (30) electrically connecting to each electrode (20) of the first set via a proximal end (22) thereof, forming a dielectric layer (40) over the first interconnect layer (30), exposing a top surface (11) of the first substrate (10) by forming a hole (50) through at least the dielectric layer (40), the hole (50) stopping at the substrate surface (11), filling the hole (50) with a sacrificial material (60), forming a composite layer (70) over the filled hole (50), said composite layer (70) comprising a first structure (71) laterally overlapping with the filled hole (50) to serve as a temporary placeholder for a microfluidic compartment (90), and a second structure (72) laterally enclosing the first structure (71) on at least two sides, or entirely enclosing the first structure (71) laterally, selectively etching at least part of the substrate (10) with respect to the first set of electrodes (20) and the filled hole (50), so as to expose the first set of electrodes (20) and a surface (61) of the filled hole (50), and removing the sacrificial material (60) and the first structure (71), thereby forming a through-hole (80) in the substrate (10) where the filled hole (50) was present and a microfluidic compartment (90) where the first structure (71) was present, said microfluidic compartment (90) being fluidically connected with the through-hole (80).

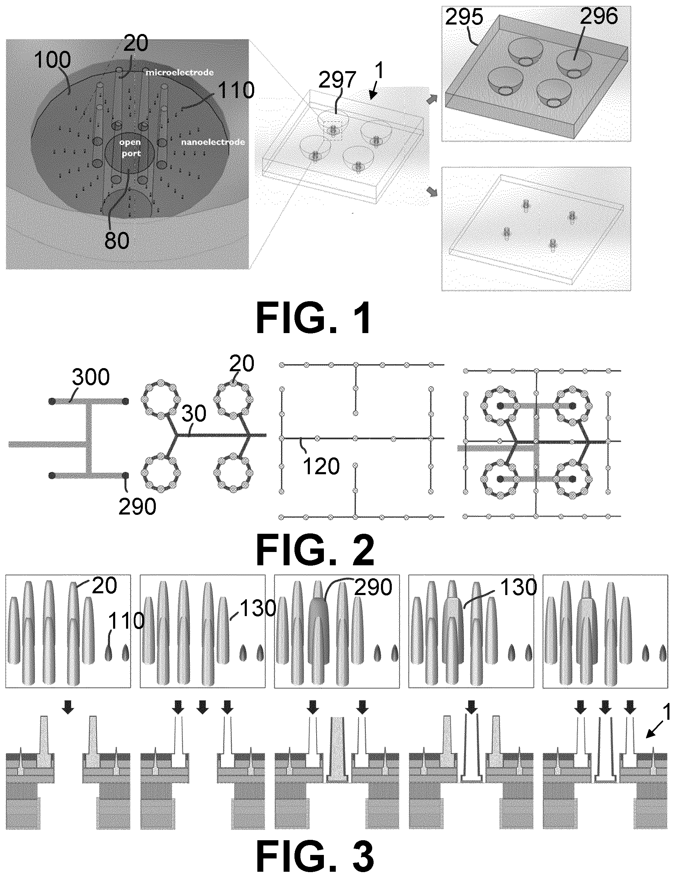

As depicted in Figure 1, in embodiments, the method may further comprise, after step vii, preferably after step viii, attaching a receiving layer (295, top right of Figure 1) on the side of the device on which the electrodes (20) of the first set are apparent, said receiving layer (295) having at least one receiving hole (296), wherein said receiving layer (295) is attached so that the receiving opening is aligned with an area of said side comprising the entrance of the through-hole (80) and the electrodes of any sets (20, 110, 290), the receiving hole (296) being dimensioned so that, when attached to said side, it forms a receiver (297) housing said electrodes (20, 110, 290) and the entrance of said through-hole (80). This receiver (297) enables the containment of the three-dimensional biological cell structure (250, not depicted in this figure) between the time it is provided over the device (1) and the time it is docked above the through-hole (80) by the electrodes (20) of the first set.

More specifically illustrated in Figure 1 is a microfluidic device (1) featuring a first (20) and a second (110) set of electrodes (micro- and nano-electrode arrays) surrounding an open port (the through hole 80) on a second substrate (a bottom silicon layer) (100). The microelectrodes (20) are depicted as cylindrical structures arranged in a circular pattern around the central open port (80), which is also circular. The nanoelectrodes (110) are shown as smaller cylindrical structures further away from the open port (80) than the microelectrodes (20), providing a dense array for enhanced biosensing capabilities. The open port (80) is centrally located with respect to the electrodes, ensuring fluid communication between the electrode compartment and the microfluidic compartment (not shown). The figure also includes an assembled chip view (central pane), showing the integration of the silicon layer comprising the electrodes with a top polymer layer that has a concave receiver (297). This concave receiver is designed to facilitate organoid docking, providing a stable environment for three-dimensional biological cell structures (250). The assembled chip is depicted with multiple concave receivers (297), each aligned with the underlying electrode arrays (20, 110) and open ports (80), indicating a modular design suitable for simultaneous multi-organoid docking and monitoring. The relative positioning of the electrodes (20, 110) and the open port (80) is advantageous for the device's functionality, allowing for precise intra- and peri-organoid electrophysiological measurements.

In embodiments, the receiving layer (295) may be a polymer layer.

In embodiments, the receiving holes (296) and the receiver (297) may have a concave shape.

In embodiments, each receiver (297) may be positioned directly over the first set of electrodes (20) forming a ring surrounding the through-hole (80), so that the three-dimensional biological cell structure (250) can be docked in the receiver (297) while remaining in fluidic communication with a microfluidic compartment (90) through the through-hole (80).

In embodiments, the receiver layer may comprise multiple receiving holes (296) arranged in an array, each receiving hole (296) aligned with all corresponding sets of electrodes (20, 110, 290) and open port (80), thereby enabling simultaneous docking and monitoring of multiple three-dimensional biological cell structures (250).

In embodiments, the first set of one or more electrodes (20) formed in the first substrate (10) may form a ring of at least three electrodes (20), wherein the top surface (11) of the first substrate (10) exposed is inside the ring, and wherein the hole (50) is laterally positioned entirely within the boundary of the ring. This configuration allows for effective docking of three-dimensional biological cell structures.

In embodiments, the at least three electrodes (20) may be from 3 to 10, more preferably from 4 to 8, yet more preferably from 5 to 7, such as 6 electrodes. This optimized number of electrodes provides balanced structural support and electrical recording capabilities.

In embodiments, the ring may have an outermost boundary having a maximal length measured parallel to the top surface (11) of the substrate (10) of from 50 to 400 micrometers, preferably from 70 to 300 micrometers, more preferably from 80 to 250 µm. This dimension ensures proper sizing for accommodating various organoid sizes.

In embodiments, the first set of electrodes (20) may be a first set of micro-electrodes. This allows for precise electrical measurements at the micro-scale.

In embodiments, the microfluidic device may be for culturing three-dimensional biological cell structures. This enables advanced biological research applications.

In embodiments, the exposed part of each electrode (20) of the first set obtained after step vii may have a height-on-width ratio of at least two. This high aspect ratio improves electrode performance and integration capabilities.

In embodiments, the exposed part of each electrode (20) of the first set obtained after step vii may have a height of from 30 to 300 µm, preferably from 100 to 290 µm, and more preferably from 125 to 280 µm, such as from 180 to 270 µm.

In embodiments, the exposed part of each electrode (20) of the first set obtained after step vii may have a width at its base of from 5 to 50 µm, preferably from 8 to 40 µm, and more preferably from 10 to 25 µm.

In embodiments, step i and step ii may comprise providing the first substrate (10) with a hard mask stack (12) and a lithographic mask (13), the lithographic mask (13) defining an interconnect design for forming the first interconnect layer (30), anisotropically etching the hard mask stack (12) by using the lithographic mask (13), thereby forming first interconnect trenches (14), providing a new mask (15) over the first interconnect trenches (14), said new mask (15) defining one or more openings (16), each opening (16) overlapping with a trench (14), wherein the openings (16) are for forming the first set of one or more electrodes (20), anisotropically etching the substrate (10) through the new mask (15) to form a mold (17) for the first set of one or more electrodes (20), and providing a conductive material (18) in the mold (17) and the trenches (14), thereby forming the first set of one or more electrodes (20) and the first interconnect layer (30) electrically connecting to each electrode (20) of the first set via a proximal end (22) thereof. This precise fabrication process ensures reliable electrode formation.

Referring to Figure 2, a schematic representation of the interconnect designs for an array of four microfluidic devices according to an embodiment is illustrated. The figure is divided into four sections, each depicting a different interconnect configuration. In section A, a third interconnect layer (300) is depicted. It is composed of a horizontal line intersecting with a vertical line, forming a T-shape. In turn, each horizontal line intersects with a horizontal line. At the ends of these last horizontal lines, circles are present, indicating four electrodes (290) of the third set, i.e., four central electrodes (290). Section B shows a first interconnect layer (30). It comprises a central horizontal line with branches extending diagonally to connect with circular formations. Each circle is composed of smaller circles (20), representing electrodes (20) of the first set. Section C features a second interconnect layer (120). It is depicted as a grid-like pattern with vertical and horizontal lines. At the intersections and endpoints of these lines, small circles are depicted, representing electrodes (110) of the second set. Section 4 combines all elements from the previous sections. The visual elements, such as lines and circles, are used to represent the various interconnect designs, highlighting the relationships and overlaps between different electrode types within the microfluidic device.

In embodiments, the new mask (15) may define a ring of at least three openings, and wherein the substrate (10) is etched anisotropically through the new mask (15) to form a ring-shaped mold for the first set of at least three electrodes (20). This approach creates a well-defined ring structure.

In embodiments, step iv may comprise providing the dielectric layer (40) with a lithographic mask defining a hole design, and anisotropically etching the dielectric layer (40) by using the lithographic mask, thereby exposing the top surface (11) of the substrate (10) by forming a hole (50) through the dielectric layer (40), the hole (50) stopping at the substrate (10) surface (11). This controlled etching process creates precise through-holes.

In embodiments, a ring of at least three electrodes (20) may be formed in step i, wherein the lithographic mask defines a hole design laterally positioned entirely within the boundary of the ring, and wherein the hole (50) formed through the dielectric layer (40) is laterally positioned entirely within the boundary of the ring. This configuration optimizes the spatial relationship between electrodes and through-hole (80).

In embodiments, step vi may comprise providing a first material layer over the filled hole (50), providing the first material layer with a lithographic mask defining the lateral extent of a temporary placeholder for a microfluidic compartment (90), anisotropically etching the first material layer by using the lithographic mask, thereby forming the first structure (71), forming a second material layer over the first structure (71), and planarizing the second material layer so as to expose a top surface of the first structure (71) and so as to form the second structure (72). This process facilitates creation of complex microfluidic structures.

In embodiments, the method may further comprise, between step vi and step vii, bonding a second substrate (100) to the composite layer (70), forming an opening (101) in the second substrate (100), said opening (101) exposing at least part of the first structure (71), and filling this opening (101) with a filling material (102), wherein step viii further comprises removing the filling material (102) after the sacrificial material (60) and the first structure (71) have been removed. This allows for multi-layered device construction.

In embodiments, the second substrate (100) may comprise a semiconductor material, preferably silicon. This enables compatibility with standard semiconductor fabrication processes.

In embodiments, the microfluidic device under construction obtained after step vi may be flipped over before performing step vii. This facilitates access to the backside for further processing.

In embodiments, the method may further comprise, between step iii and iv, forming a second set of one or more electrodes (110) in the substrate (10), the electrodes (110) of the second set having a different height than the electrodes (20) of the first set, each electrode (110) of the second set having a distal end (111) buried in the substrate (10), and forming a second interconnect layer (120) electrically connecting to each electrode (110) of the second set via a proximal end (112) thereof, and wherein step vii is also performed selectively with respect to the second set of electrodes (110), so as to expose at least the distal end (111) of the electrodes (110) of the second set in addition to exposing the first set of electrodes (20) and the surface (61) of the filled hole (50). This multi-height electrode configuration allows for both intra- and peri-organoid sensing.

In embodiments, the exposed height of the electrodes (110) of the second set after step vii may be smaller than the exposed height of the electrodes (20) of the first set. This dimensional difference provides complementary sensing capabilities.

In embodiments, the exposed height of the electrodes of the second set obtained after step vii may be from 1 µm to 30 µm, preferably from 3 to 25 µm, more preferably from 5 to 20 µm, yet more preferably from 8 to 15 µm.

In embodiments, the exposed part of each electrode (20) of the second set obtained after step vii may have a width at its base of from 0.100 to 2 µm, preferably from 0.200 to 0.900 µm, and more preferably from 0.300 to 0.700 µm.In embodiments, the ratio of the exposed part of the electrodes of the first set to the electrodes of the second set may be from 1 to 60, preferably from 10 to 40, more preferably from 15 to 30.

In embodiments, the electrodes (110) of the second set may be nanoelectrodes. These nanoelectrodes enable intracellular measurements.

In embodiments, the method may further comprise, between steps v and vi, forming a third set of one or more electrodes (290) in the sacrificial material (60) and in the first substrate (10), each electrode (290) of said third set having a distal end (292) buried in the substrate (10), having a proximal end (291) laterally surrounded by the sacrificial material (60), and forming a third interconnect layer (300) electrically connecting each electrode (290) of the third set via their proximal end (291). This center electrode (i.e., electrode of the third set) enables additional functionality such as electroporation.

In embodiments, the third set (290) may consist of a single electrode (290). This central electrode (290) provides a focused electrical field.

In embodiments, at least one electrode among the first, second, and third set may be formed to be hollow, the method comprising: (a) forming said at least one electrode with a conductive outer shell (130) and a sacrificial inner core (131), (b) forming a corresponding interconnect structure (140) that comprises a conductive outer shell (141) and a sacrificial inner core (142), wherein the conductive outer shell (130) of the at least one electrode is electrically connected to the conductive outer shell (141) of the at least one interconnect structure (140) via a proximal end of said at least one electrode, and wherein the inner core (131) of said at least one electrode is in direct physical contact with the inner core (142) of said interconnect structure (140) via a proximal end of said at least one electrode, (c) selectively removing a distal portion (132) of the conductive outer shell (130) of said at least one electrode, and (d) subsequently removing the sacrificial inner cores (131, 142) of said at least one electrode and the corresponding interconnect structure (140), thereby resulting in a hollow electrode (20) with an open distal end that is fluidically connected to a hollow interconnect structure (143) at its proximal end. This hollow electrode configuration enables molecular delivery capabilities.

Figures 9 to 13 show examples of embodiments of the first aspect. They correspond to the five designs of Figure 3.

As illustrated in Figure 3, schematic representations of five design configurations of the microfluidic device (1) are shown, each depicting different arrangements of hollow or non-hollow electrodes of the first set (20), second set (110), and third set (290) along with a through-hole (80). The designs will be referred to as DESIGN 1, DESIGN 2, and DESIGN 3 (with three options). Each design features an arrangement of electrodes of the first set (20) and second set (110), with the through-hole (80) centrally located. In DESIGN 1, the electrodes of the first set (20) are non-hollow, and the through-hole (80) is indicated by an arrow. DESIGN 2 introduces hollow electrodes (20) of the first set with conductive outer shells (130), as shown by arrows pointing to the hollow structures. DESIGN 3 Option 1 includes a central electrode of the third set (290) surrounded by non-hollow electrodes of the first set (20). Option 2 features a hollow electrode (290) of the third set at the center surrounded by non-hollow electrodes (20) of the first set, while Option 3 presents both the central electrode (290) of the third set and surrounding electrodes of the first set (20) as hollow electrodes. The cross-sectional views below each design show the structural layers, highlighting the variations in electrode configurations for culturing three-dimensional biological cell structures (250).
Referring to Figure 9, a process flow for forming the first design configuration of a microfluidic device (1) is illustrated. The sequence begins with 1. providing the first substrate (10) with a hard mask stack (12) and a lithographic mask (13), the lithographic mask (13) defining an interconnect design for forming the first interconnect layer (30), 2. anisotropically etching the hard mask stack (12) by using the lithographic mask (13), thereby forming first interconnect trenches (14), 3. providing a new mask (15) over the first interconnect trenches (14), said new mask (15) defining one or more openings (16), each opening (16) overlapping with a trench (14), wherein the openings (16) are for forming the first set of one or more electrodes (20), 4. anisotropically etching the substrate (10) through the new mask (15) to form a mold (17) for the first set of one or more electrodes (20), and 5. providing a conductive material (18) (first a conductive barrier layer, then a conductive filler) in the mold (17) and the trenches (14), followed by chemical mechanical polishing (CMP), thereby forming by dual damascene the first set of one or more electrodes (20) and the first interconnect layer (30) electrically connecting to each electrode (20) of the first set via a proximal end (22) thereof. This fabrication process ensures reliable electrode formation. This is followed by 6. forming a dielectric layer (e.g., a hard mask stack 40) over the first interconnect layer (30), providing the hard mask stack (40) with a lithographic mask (41), the lithographic mask (41) defining an interconnect design for forming the second interconnect layer (120), 7. anisotropically etching the hard mask stack (40) by using the lithographic mask (41), thereby forming second interconnect trenches (42), 8. providing a new mask (43) over the first interconnect trenches (42), said new mask (43) defining one or more openings (44), each opening (44) overlapping with a trench (42), wherein the openings (44) are for forming the second set of one or more electrodes (110), 9. anisotropically etching the substrate (10) through the new mask (43) to form a mold (45) for the second set of one or more electrodes (110), and 10. providing a conductive material (18) (first a conductive barrier layer, then a conductive filler) in the mold (45) and the trenches (42), followed by chemical mechanical polishing (CMP), thereby forming by dual damascene the second set of one or more electrodes (110) and the second interconnect layer (120) electrically connecting to each electrode (110) of the second set via a proximal end (112) thereof. This is followed by 11. providing a dielectric layer (a hard mask stack 121) with a lithographic mask (122) defining a hole design, and 12. anisotropically etching the dielectric layer (121) by using the lithographic mask (122), thereby exposing the top surface (11) of the substrate (10) by forming a hole (50) through the dielectric layers (121, 40, 12), the hole stopping at the substrate surface (11). This controlled etching process creates precise through-holes.
13. Next, the hole (50) is filled with a sacrificial material (e.g., silicon dioxide, 60) and CMP, 14. providing a first material layer (e.g., amorphous silicon, 62) over the filled hole (50), providing the first material layer (62) with a lithographic mask (63) defining the lateral extent of a temporary placeholder (71) for a microfluidic compartment (90), 15. anisotropically etching the first material layer (62) by using the lithographic mask (63), thereby forming the first structure (71), 16. forming a second material layer (e.g., silicon nitride, 64) over the first structure (71), and 17. planarizing the second material layer by CMP so as to expose a top surface of the first structure (71) and so as to form the second structure (72). This process facilitates the creation of complex microfluidic structures. 18. bonding silicon dioxide deposition, 19. alignment to the second substrate (100), 20. silicon dioxide wafer bonding, 21. lithography and 22. etch to form an opening (101) in the second substrate (100) and 23. filling with filling material (102), 24. wafer flip, 25. silicon substrate CMP, 26. substrate total or partial removal, 27. removal of the sacrificial material (60) to form the through-hole (80), 28. removal of the first structure (71) to form the microfluidic compartment (90), and finally 29. the removal of the filling material (102) to form the microfluidic compartment (200). This detailed process flow results in the formation of a microfluidic device (1) with integrated first set of electrodes (20) and second set of electrodes (110), facilitating advanced functionalities such as three-dimensional biological cell structure docking and vascularization.

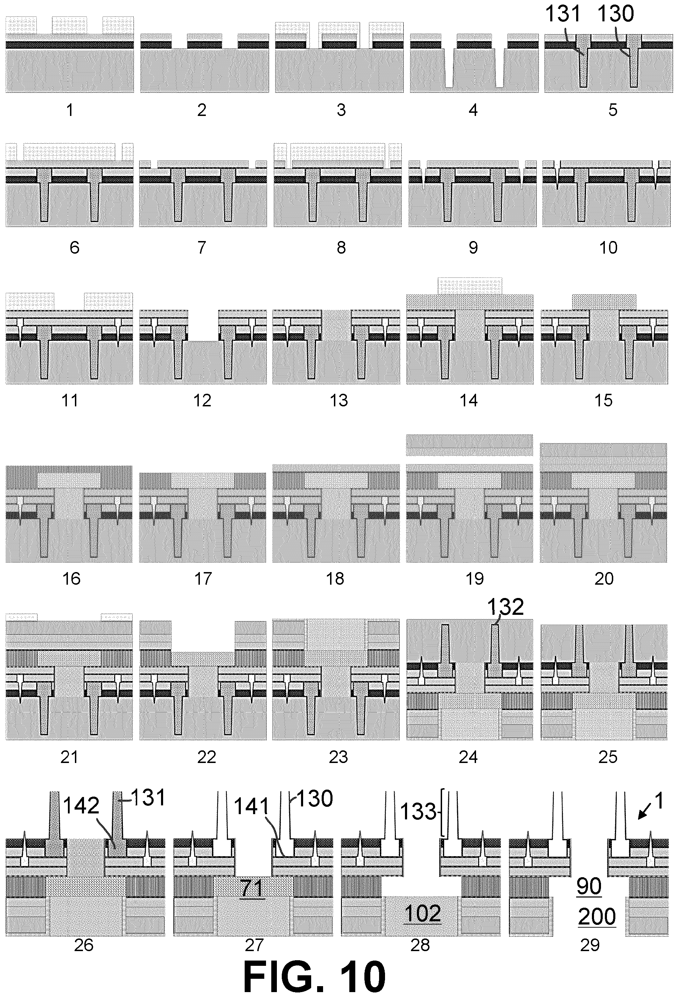

As shown in Figure 10, a process flow diagram for forming the second design configuration of the microfluidic device (1) with hollow electrodes of the first set (20) is presented. The diagram is composed of a series of sequential steps, beginning with the same steps 1 to 4 as in Figure 9. The subsequent steps involve 5. the deposition of a conductive outer shell (e.g., TiN, 130) with sacrificial inner core (e.g., SiO₂, 131) and CMP to form the first set of electrodes (20) and first interconnect layer (30), steps 6 to 24 are identical to Figure 9. In step 25, CMP is performed on the substrate until the sacrificial inner core of the electrodes of the first set is revealed by removing a distal portion (132) of the conductive outer shell (130). 26. At least partial removal of the substrate (10) material to expose the underlying features, 27. removal of the sacrificial material (60) and the sacrificial inner cores (131, 142), 28. removal of the first structure (71), and 29. removal of the filling material (102), resulting in the formation of hollow electrodes (20) of the first set, the through-hole (80) and the microfluidic compartment (90, 200).

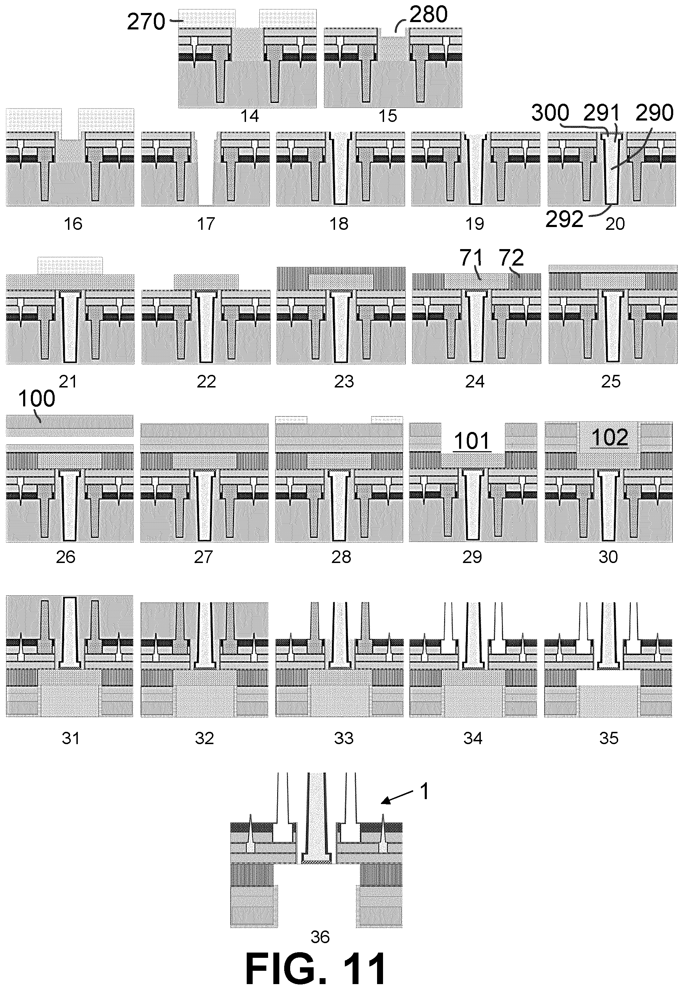

Referring to Figure 11, the process flow for forming the third design configuration (option 1) of the microfluidic device (1) with a non-hollow electrode of the third set (290) at the center of the device is shown. The process begins with the same steps 1-13 as for Figure 10. The process continues with steps 14 to 18 for the creation of the third set of electrodes (here a single central electrode 290) within the sacrificial material (60) and the substrate (10), connecting to a third interconnect layer (300). In step 14, a lithographic mask (270) is formed over the device under construction. This mask (270) has openings for forming a third set of interconnect trenches (280). In step 15, the interconnect trenches (280) are formed by anisotropic etching using the mask (270). In step 16, a further lithographic mask (285) is formed over the device under construction. This mask (285) has openings for forming the mold(s) for the electrodes (290) of the third set. In step 17, the molds are formed by anisotropic etching using the mask (285). In step 18, a conductive material (first a liner, then a conductive filler) is deposited so as to fill the mold(s) and the interconnect trenches (280) in a dual damascene process, followed by CMP. At this stage, a step 19 of recessing a top portion of the conductive material, and a step 20 of forming a plug in the void left by the recess, can be performed (depicted). Subsequent steps are similar to Figures 9 and 10 and include the formation of the first structure (71) through 21. lithography and 22. etching, 23-24. deposition of, e.g., silicon nitride to form the second structure (72), 25-27. bonding to the second substrate (100), 28-29. creation of an opening (101) filled in step 30 with filling material (102), 31. wafer flip, 32. substrate thinning until the sacrificial inner core of the electrodes of the first set are exposed, and 33. At least partial substrate removal, and 34-36 final removal of the sacrificial material (60), the first structure (71), and the filling material (102) to reveal the through-hole (80), and the microfluidic compartment (90).

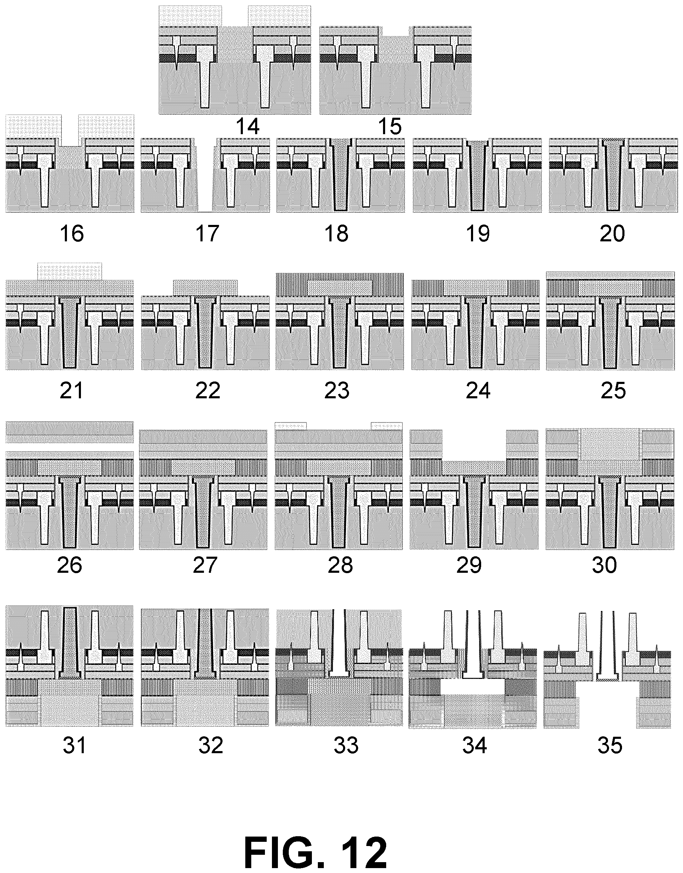

As illustrated in Figure 12, the process flow for forming the third design configuration (option 2) of the microfluidic device (1) with a hollow electrode of the third set (290) at the center surrounded by non-hollow electrodes of the first set (20) is presented. The process begins with the same steps 1-13 as for Figure 9. It then follows the same steps 14-17 as for Figure 11. Then, step 18 is performed to deposit conductive material (18) and CMP is performed to form the first set of electrodes (20) and first interconnect layer (30). At this stage, a step 19 of recessing a top portion of the conductive material, and a step 20 of forming a plug in the void left by the recess, can be performed (depicted). This is followed by the same steps 21 to 31 as for Figure 11. In step 32, CMP is performed until the inner filling of the central electrode (290) of the third set is exposed. Then, 33-35. at least part of the substrate (10) and all sacrificial materials are removed to form a hollow electrode (290) of the third set, the through-hole (80), and the microfluidic compartment (90).

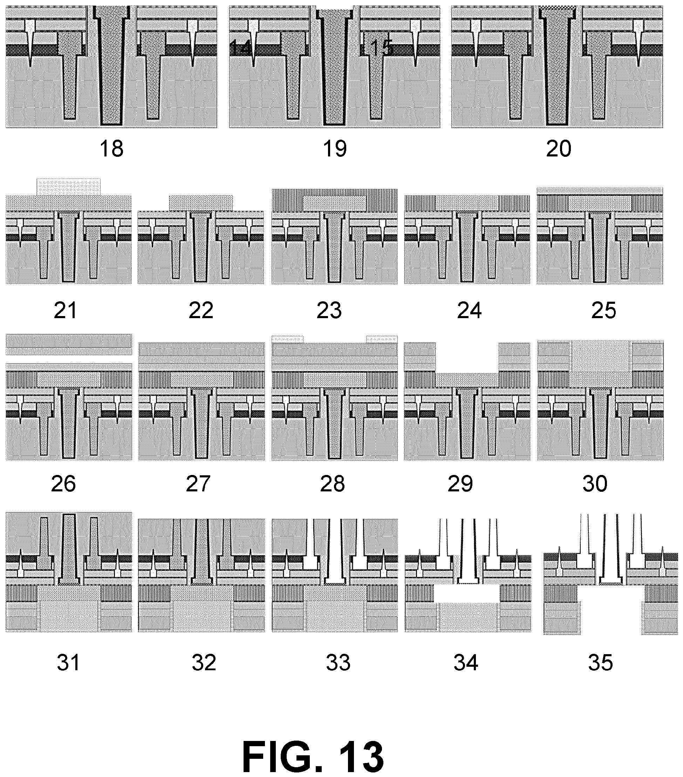

Referring to Figure 13, the process flow for forming the third design configuration (option 3) of the microfluidic device (1) with a hollow electrode of the third set (290) at the center surrounded by hollow electrodes of the first set (20) is presented. Steps 1-17 are as described for Figure 11. Steps 18-32 are as described for Figure 12. In step 33, CMP is performed to expose the sacrificial inner core of the electrodes of the first and third set, followed by 34-35. Removal of the substrate and of all sacrificial materials including inner cores to form hollow electrodes (20, 290) of both the first and third sets, the through-hole (80), and the microfluidic compartment (90), thereby completing the device with both hollow microelectrodes and a hollow central electrode suitable for advanced organoid culturing applications.

In embodiments, the electrodes of the first and third sets are of the same height. This facilitate the fabrication of hollow electrodes, especially hollow electrodes of the first set.

In embodiments, the exposed part of each electrode (20) of the third set obtained after step vii may have a height of from 30 to 300 µm, preferably from 100 to 290 µm, and more preferably from 125 to 280 µm, such as from 180 to 270 µm.

In embodiments, the exposed part of each electrode (20) of the third set obtained after step vii may have a width at its base of from 5 to 50 µm, preferably from 8 to 40 µm, and more preferably from 10 to 25 µm.

In a second aspect, the present invention relates to a microfluidic device, comprising an electrode compartment (210) and a microfluidic compartment (200), the electrode compartment (210) comprising a first set of one or more electrodes (20) extending from a proximal end (22) closest to the microfluidic compartment (200) to a distal end (21) farthest from the microfluidic compartment (200), a separation layer (230) between the microfluidic compartment (200) and the electrode compartment (210), the separation layer (230) comprising a first interconnect layer (30) electrically connected to each electrode (20) of the first set at the proximal end (22) thereof; a dielectric layer (40) between the first interconnect layer (30) and the microfluidic compartment (200); and a through-hole (80) extending through the separation layer (230), thereby ensuring fluid communication between the electrode compartment (210) and the microfluidic compartment (200).

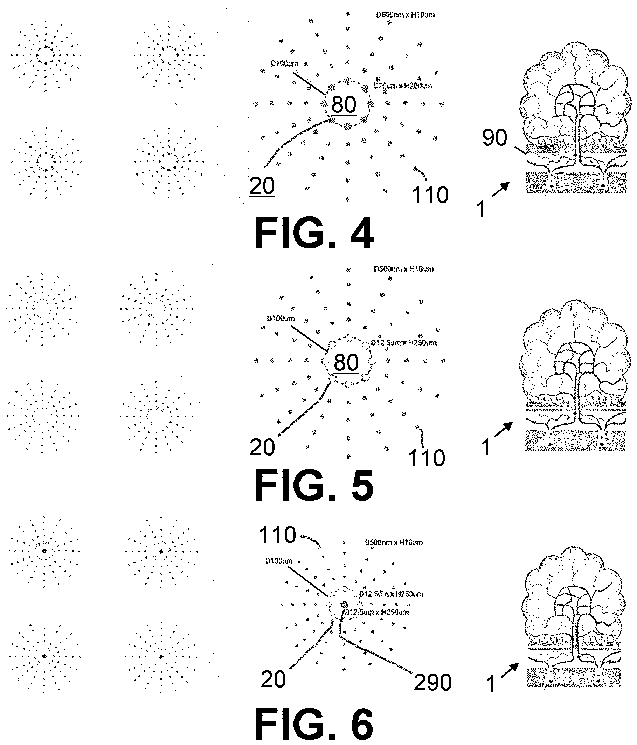

As illustrated in Figure 4, the first design configuration of the microfluidic device (1) is shown featuring a perforated substrate (10) with electrodes of the first set (20) and second set (110), and through-holes (80) for three-dimensional biological cell structure (250) docking and electrophysiological recording. The left pane shows a top view of multiple circular arrangements of electrodes, each surrounding a through-hole (80). The electrodes are depicted as small dots, with a central through-hole (80) represented by a larger circle. The central pane provides a magnified view of one such arrangement, detailing the dimensions: the central through-hole (80) has a diameter of 100 micrometers, surrounded by electrodes of the first set (20) with a diameter of 20 micrometers and a height of 200 micrometers. Electrodes of the second set (110) are depicted as smaller dots with dimensions of 500 nanometers in diameter and 10 micrometers in height. The rightmost pane shows a cross-sectional schematic of the microfluidic device (here a bio-docking station), illustrating the intra-organoid microcirculation through the through-hole (80) and the microfluidic compartment (90) beneath, with in-flow and out-flow channels facilitating fluid communication. This configuration supports the docking of three-dimensional biological cell structures (250) and vascularization, enabling detailed electrophysiological monitoring.

In embodiments, a microfluidic system may comprise a substrate with a through-hole (80) ensuring fluid communication between an electrode compartment (210) and a microfluidic compartment (200), and at least one fluidic circuit including a dedicated inflow channel and a dedicated outflow channel arranged so as to provide directional fluid flow across the through-hole (80). This promotes vascularization or nutrient supply to a three-dimensional biological cell structure (250) positioned above the through-hole (80).

In embodiments, the fluidic circuit may be configured so that the through-hole (80) lies laterally between the inflow channel and the outflow channel, enabling controlled perfusion around or through the three-dimensional biological cell structure. This allows enhanced circulation of nutrients, waste removal, or vascular in-growth.

In embodiments, the inflow and outflow channels may each comprise adjustable flow regulators connected to a fluid control unit (310), and the electrode compartment may further comprise at least one hollow electrode open to the fluidic circuit for delivery of targeted agents into the three-dimensional biological cell structure.

In embodiments, each electrode (30) of the first set may have an exposed height-to-width ratio of at least two. This high aspect ratio provides improved electrical performance.

In embodiments, the exposed part of each electrode (20) of the first set may have a height of from 30 to 300 µm, preferably from 100 to 290 µm, and more preferably from 125 to 280 µm, such as from 180 to 270 µm.

In embodiments, the exposed part of each electrode (20) of the first set may have a width at its base of from 5 to 50 µm, preferably from 8 to 40 µm, and more preferably from 10 to 25 µm.

In embodiments, the microfluidic device and the electrode compartment (210) may be for culturing three-dimensional biological cell structures. This configuration supports complex biological research applications.

In embodiments, the first set of one or more electrodes (30) may be a ring of at least three electrodes (30) and wherein the through-hole (80) is laterally positioned entirely within the boundary of the ring. This arrangement creates an effective docking station for organoids.

In embodiments, the at least three electrodes (30) of the first set may be from 3 to 10, more preferably from 4 to 8, yet more preferably from 5 to 7, such as 6. This optimized number balances structural support and sensing capabilities.

In embodiments, the ring may have an outermost boundary having a maximal length measured parallel to the top surface of the substrate of from 50 to 400 micrometers, preferably from 70 to 300 micrometers, more preferably from 80 to 250 µm. This sizing accommodates various organoid dimensions.

In embodiments, the first set of one or more electrodes (30) may be a first set of one or more micro-electrodes. This allows for precise electrical measurements at the micro scale.

In embodiments, the microfluidic device may further comprise a second set of one or more electrodes (110) extending from a proximal end (112) closest to the microfluidic compartment (200) to a distal end (111) farthest from the microfluidic compartment (200), the electrodes (110) of the second set having a different height (e.g., a smaller height) than the electrodes (30) of the first set, and a second interconnect layer (120) in the separation layer (230), electrically connecting to the electrodes (110) of the second set via a proximal end (112) thereof. This dual-height configuration enables complementary sensing capabilities.

In embodiments, the exposed height of the electrodes of the second set may be from 1 µm to 30 µm, preferably from 3 to 25 µm, more preferably from 5 to 20 µm, yet more preferably from 8 to 15 µm.

In embodiments, the exposed part of each electrode (20) of the second set may have a width at its base of from 0.100 to 2 µm, preferably from 0.200 to 0.900 µm, and more preferably from 0.300 to 0.700 µm.

As shown in Figure 5, the second design configuration of the microfluidic device (1) is presented featuring a perforated substrate (10) with hollow electrodes (20) of the first set and fluidic channels for targeted molecule delivery. The figure is divided into three panes. The left pane shows a top view of four circular arrays of hollow electrodes (20), each array being arranged in a ring pattern. Each array consists of a central ring of hollow electrodes (20) surrounded by a larger field of electrodes of the second set (110). The central ring is labeled with dimensions, indicating a diameter of 100 micrometers and individual hollow electrode dimensions of 12.5 micrometers in diameter and 250 micrometers in height. The middle pane provides a magnified view of one of the circular arrays, highlighting the detailed arrangement of the hollow electrodes (20) with their conductive outer shells (130). The surrounding electrodes of the second set (110) are labeled with dimensions of 500 nanometers in diameter and 10 micrometers in height. The pane section is a schematic representation of the intra-organoid microcirculation system. It illustrates the microfluidic device (here a bio-docking station) with hollow electrodes (20) integrated with a through-hole (80) and fluidic channel system. The diagram shows the microfluidic compartment (90) beneath the bio-docking station, with labeled in-flow and out-flow channels, demonstrating the fluidic connectivity for delivering molecules into the three-dimensional biological cell structure (250).

Referring to Figure 6, the third design configuration (option 1) of the microfluidic device (1) is shown featuring an electrode of the third set (290) at the center of the microfluidic device (here a bio-docking station) for electroporation of genetic material. The figure is divided into three panes. The left pane shows a top view of multiple circular arrays, each comprising a central electrode of the third set (290) surrounded by a ring of electrodes of the first set (20) and concentric rings of electrodes of the second set. The middle section provides a magnified view of one circular array, detailing the dimensions: the central electrode of the third set (290) has a diameter of 12.5 micrometers and a height of 250 micrometers, while the surrounding electrodes of the first set (20) have a diameter of 12.5 micrometers and a height of 250 micrometers, with electrodes of the second set (110) having dimensions of 500 nanometers in diameter and 10 micrometers in height. The right pane presents a schematic cross-sectional view of the device, illustrating the intra-organoid microcirculation facilitated by the microfluidic device (or bio-docking station), through-hole (80), and electrode of the third set (290), along with labeled in-flow and out-flow channels indicating the fluidic pathways within the microfluidic compartment (90). This configuration enables precise electroporation within three-dimensional biological cell structures (250).

In embodiments, the exposed part of each electrode (20) of the third set may have a height of from 30 to 300 µm, preferably from 100 to 290 µm, and more preferably from 125 to 280 µm, such as from 180 to 270 µm.

In embodiments, the exposed part of each electrode (20) of the third set may have a width at its base of from 5 to 50 µm, preferably from 8 to 40 µm, and more preferably from 10 to 25 µm.

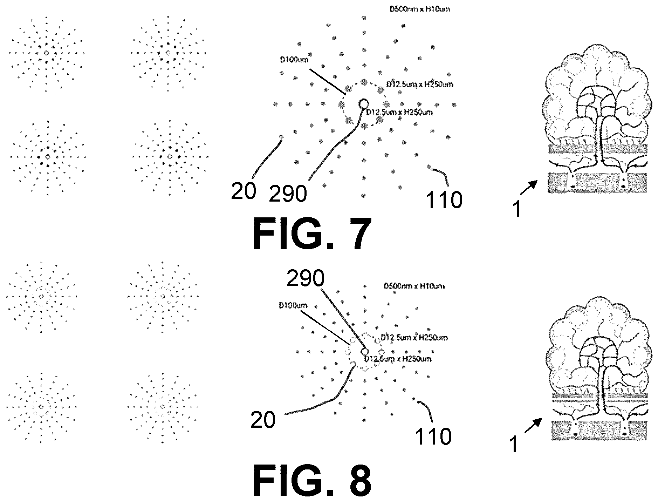

As illustrated in Figure 7, the third design configuration (option 2) of the microfluidic device (1) is shown featuring a hollow electrode (290) of the third set at the center for comparative intra-organoid genetic reprogramming. The figure is divided into three panes. The left pane shows a top view of multiple circular arrays, each consisting of a central hollow electrode (290) of the third set surrounded by a ring of non-hollow electrodes of the first set (20). The entire array is encompassed by a larger field of electrodes of the second set (110). The middle pane provides a detailed view of a single array, where the central hollow electrode (290) of the third set is marked with dimensions 12.5 micrometers in diameter and 250 micrometers in height, and is surrounded by a ring of electrodes of the first set (20), each with dimensions 12.5 micrometers in diameter and 250 micrometers in height, the entire ring having a diameter of 100 micrometers. The electrodes of the second set (110) have dimensions of 500 nanometers in diameter and 10 micrometers in height. The right section is a schematic representation of the microfluidic device (or bio-docking station), illustrating the intra-organoid microcirculation facilitated by the hollow electrode (290) of the third set, and showing the integration with the through-hole (80) and fluidic channels, highlighting the microfluidic compartment (90) and the in-flow and out-flow channels. This configuration enables precise genetic reprogramming within three-dimensional biological cell structures (250) by utilizing the hollow electrode (20) of the third set for targeted delivery and electroporation.

As shown in Figure 8, the third design configuration (option 3) of the microfluidic device (1) is presented featuring a hollow electrode (290) of the third set at the center surrounded by hollow electrodes (20) of the first set for comparative intra-organoid electroporation of genetic material. The figure is divided into three panes. The left pane shows a top view of multiple circular arrays, each consisting of a central hollow electrode (290) of the third set, indicated by its hollow center, surrounded by a ring of hollow electrodes (20) of the first set. The central hollow electrode (290) of the third set is labeled with dimensions 12.5 micrometers in diameter and 250 micrometers in height, and the surrounding hollow electrodes (20) of the first set are similarly dimensioned, with the entire array enclosed within a boundary of 100 micrometers in diameter. Electrodes of the second set (110) have dimensions of 500 nanometers in diameter and 10 micrometers in height. The right pane presents a cross-sectional schematic of the microfluidic device (1), depicting a three-dimensional biological cell structure (250) with intra-organoid microcirculation positioned above a microfluidic device (or bio-docking station) that includes the hollow electrodes (20, 290) of both the first and third sets, along with the through-hole (80) and fluidic channels labeled as "in-flow channel" and "out-flow channel" indicating the direction of fluid movement through the microfluidic compartment (90). This configuration facilitates comparative intra-organoid electroporation, allowing for the delivery of genetic material into the three-dimensional biological cell structure (250), with the hollow electrodes (20, 290) and fluidic channels enabling precise control of the microenvironment.

In embodiments, at least one electrode among the first (30), second (110), and third (290) set may be hollow and comprises a conductive outer shell (130) forming the outer surface of the electrode, wherein the interconnect structure is hollow and has a conductive outer shell (141), and wherein a proximal end of said hollow electrode is fluidically connected to the hollow interconnect structure. This hollow structure enables molecular delivery. Examples of thickness of the conductive outer shell in the case of electrodes of the first and third sets are from 0.5 to 2µm.

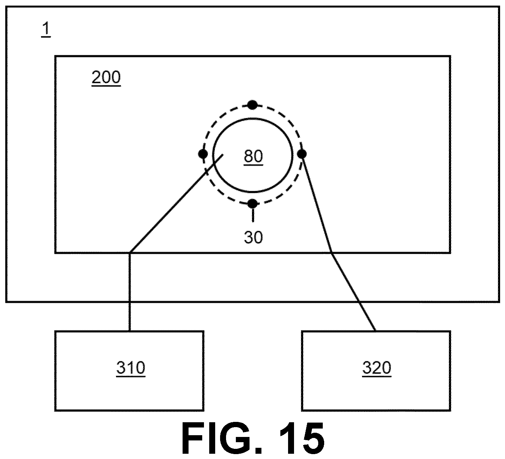

In a third aspect, illustrated in Figure 15, the present invention relates to a system for culturing and monitoring three-dimensional biological cell structures, comprising the microfluidic device according to any embodiments of the second aspect; a fluid control unit (310) configured to control fluid flow through the microfluidic compartment (200) and the through-hole (80), and an electronic measurement unit (320) configured to receive and process electrical signals from at least the first set of one or more electrodes (30) and from the second and/or third set if present.

It is to be understood that although preferred embodiments, specific constructions and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks. Steps may be added or deleted to methods described within the scope of the present invention.

### List of Reference Numbers

(1) - microfluidic device
(10) - first substrate
(11) - top surface of the first substrate
(12) - hard mask stack
(13) - lithographic mask
(14) - first interconnect trenches
(15) - new mask
(16) - openings
(17) - mold
(18) - conductive material
(20) - first set of one or more electrodes
(21) - distal end of electrode of the first set
(22) - proximal end of electrode of the first set
(23) - ring of at least three electrodes
(30) - first interconnect layer
(40) - dielectric layer
(41) - lithographic mask
(42) - second interconnect trenches
(43) - new mask
(44) - opening
(45) - mold
(50) - hole
(60) - sacrificial material
(61) - surface of the filled hole
(62) - first material layer
(63) - lithographic mask
(64) - second material layer
(70) - composite layer
(71) - first structure, temporary placeholder
(72) - second structure
(80) - through-hole
(90) - microfluidic compartment
(100) - second substrate
(101) - opening in the second substrate
(102) - filling material
(110) - second set of one or more electrodes
(111) - distal end of electrode of the second set
(112) - proximal end of electrode of the second set
(120) - second interconnect layer
(121) - dielectric layer
(122) - lithographic mask
(130) - conductive outer shell of electrode of the first or third set
(131) - sacrificial inner core of electrode of the first set
(132) - distal portion of the conductive outer shell of electrode of the first set
(140) - interconnect structure
(141) - conductive outer shell of interconnect structure
(142) - sacrificial inner core of interconnect structure
(143) - hollow interconnect structure
(200) - microfluidic compartment
(210) - electrode compartment
(230) - separation layer
(250) - three-dimensional biological cell structure
(270) - lithographic mask
(280) - third interconnect trenches
(285) - further lithographic mask
(290) - third set of one or more electrodes
(291) - proximal end of electrode of the third set
(292) - distal end of electrode of the third set
(295) - receiving layer
(296) - receiving hole
(297) - receiver
(300) - third interconnect layer
(310) - fluid control unit
(320) - electronic measurement unit

## Claims

1. A method for forming a microfluidic device (1), comprising:
i. providing a first substrate (10) and forming within it a first set of one or more electrodes (20), each electrode (20) having a distal end (21) buried in the substrate (10),
ii. forming a first interconnect layer (30) electrically connecting to each electrode (20) of the first set via a proximal end (22) thereof,
iii. forming a dielectric layer (40) over the first interconnect layer (30),
iv. exposing a top surface (11) of the first substrate (10) by forming a hole (50) through at least the dielectric layer (40), the hole (50) stopping at the substrate (10) surface (11),
v. filling the hole (50) with a sacrificial material (60),
vi. forming a composite layer (70) over the filled hole (50), said composite layer (70) comprising:
a. a first structure (71) laterally overlapping with the filled hole (50) to serve as a temporary placeholder for a microfluidic compartment (90), and
b. a second structure (72) laterally enclosing the first structure (71) on at least two sides, or entirely enclosing the first structure (71) laterally,
vii. selectively etching at least part of the substrate (10) with respect to the first set of electrodes (20) and the filled hole (50), so as to expose:
a. the first set of electrodes (20), and
b. a surface (61) of the filled hole (50), and
viii. removing the sacrificial material (60) and the first structure (71), thereby forming a through-hole (80) in the substrate (10) where the filled hole (50) was present and a microfluidic compartment (90) where the first structure (71) was present, said microfluidic compartment (90) being fluidically connected with the through-hole (80).

2. The method according to claim 1, wherein the first set one or more electrodes (20) formed in the first substrate (10) in step i. forms a ring (23) of at least three electrodes (20), wherein the top surface (11) of the first substrate (10) exposed in step iv. is inside the ring (23), and wherein the hole (50) is laterally positioned entirely within the boundary of the ring (23).

3. The method according to claim 1 or claim 2, wherein step i and step ii comprise:
o providing the first substrate (10) with a hard mask stack (12) and a lithographic mask (13), the lithographic mask (13) defining an interconnect design for forming the first interconnect layer (30),
o anisotropically etching the hard mask stack (12) by using the lithographic mask (13), thereby forming first interconnect trenches (14),
o providing a new mask (15) over the first interconnect trenches (14), said new mask (15) defining one or more openings (16), each opening (16) overlapping with a trench (14), wherein the openings (16) are for forming the first set of one or more electrodes (20),
∘ anisotropically etching the substrate (10) through the new mask (15) to form a mold (17) for the first set of one or more electrode (20), and
∘ providing a conductive material (18) in the mold (17) and the trenches (14), thereby forming the first set of one or more electrodes (20) and the first interconnect layer (30) electrically connecting to each electrode (20) of the first set via a proximal end (22) thereof.

4. The method according to any one of the preceding claims, further comprising, between step vi and step vii:
o bonding a second substrate (100) to the composite layer (70),
o forming an opening (101) in the second substrate (100), said opening (101) exposing at least part of the first structure (71), and
∘ filling this opening (101) with a filling material (102),
wherein step viii further comprises removing the filling material (102) after the sacrificial material (60) and the first structure (71) have been removed.

5. The method according to any one of the preceding claims, further comprising, between step iii and iv:
o forming a second set of one or more electrodes (110) in the substrate (10), the electrodes (110) of the second set having a different height than the electrodes (20) of the first set, each electrode (110) of the second set having a distal end (111) buried in the substrate (10), and
∘ forming a second interconnect layer (120) electrically connecting to each electrode (110) of the second set via a proximal end (112) thereof,
and wherein step vii is also performed selectively with respect to the second set of electrodes (110), so as to expose at least the distal end (111) of the electrodes (110) of the second set in addition to exposing the first set of one or more electrodes (20) and the surface (61) of the filled hole (50).

6. The method according to any one of claims 1 to 5, further comprising, between steps v and vi:
- forming a third set of one or more electrodes (290) in the sacrificial material (60) and in the first substrate (10), each electrode (290) of said third set:
- having a distal end (291) buried in the substrate (10),
- having a proximal end (292) laterally surrounded by the sacrificial material (60), and
- forming a third interconnect layer (300) electrically connecting each electrode (290) of the third set via their proximal end (292).

7. The method according to any one of the preceding claims, wherein at least one electrode among the first, second, and third set is formed to be hollow, the method comprising: (a) forming said at least one electrode with a conductive outer shell (130) and a sacrificial inner core (131), (b) forming a corresponding interconnect structure (140) that comprises a conductive outer shell (141) and a sacrificial inner core (142), wherein the conductive outer shell (130) of the at least one electrode is electrically connected to the conductive outer shell (141) of the at least one interconnect structure (140) via a proximal end of said at least one electrode, and wherein the inner core (131) of said at least one electrode is in direct physical contact with the inner core (142) of said interconnect structure (140) via a proximal end of said at least one electrode, (c) selectively removing a distal portion (132) of the conductive outer shell (130) of said at least one electrode, and (d) subsequently removing the sacrificial inner cores (131, 142) of said at least one electrode and the corresponding interconnect structure (140), thereby resulting in a hollow electrode (20) with an open distal end that is fluidically connected to a hollow interconnect structure (143) at its proximal end.

8. A microfluidic device (1), comprising:
a) a microfluidic compartment (200),
b) an electrode compartment (210) comprising a first set of one or more electrodes (30) extending from a proximal end (22) closest to the microfluidic compartment (200) to a distal end (21) farthest from the microfluidic compartment (200),
c) a separation layer (230) between the microfluidic compartment (200) and the electrode compartment (210), the separation layer (230) comprising:
i) a first interconnect layer (30) electrically connected to each electrode (30) of the first set at the proximal end (22) thereof,
ii) a dielectric layer (40) between the first interconnect layer (30) and the microfluidic compartment (200); and
d) a through-hole (80) extending through the separation layer (230), thereby ensuring fluid communication between the electrode compartment (210) and the microfluidic compartment (200).

9. The microfluidic device (1) according to claim 8, wherein the microfluidic device (1) and the electrode compartment (210) are for culturing three-dimensional biological cell structures (250) .

10. The microfluidic device (1) according to claim 8 or claim 9, wherein the first set of one or more electrodes (30) is a ring of at least three electrodes (30) and wherein the through-hole (80) is laterally positioned entirely within the boundary of the ring.

11. The device (1) according to any one of claims 8 to 10, further comprising:
o a second set of one or more electrodes (110) extending from a proximal end (112) closest to the microfluidic compartment (200) to a distal end (111) farthest from the microfluidic compartment (200), the electrodes (110) of the second set having a different height than the electrodes (30) of the first set, and
o a second interconnect layer (120) in the separation layer (230), electrically connecting to the electrodes (110) of the second set via a proximal end (112) thereof.

12. The microfluidic device (1) according to any one of claims 8 to 11, wherein the electrode compartment (210) further comprises:
o a third set of one or more electrodes (290), each electrode (290) of the third set:
o extending from a proximal end (291) closest to the microfluidic compartment (200) to a distal end (292) farthest from the microfluidic compartment (200),
o being laterally surrounded by the first set of one or more electrodes (30) if the first set comprises a plurality of electrodes (30),
o being in the through-hole (80) while being laterally spaced apart from the inner walls of the through-hole (80), thereby ensuring fluidic connection between the microfluidic compartment (200) and the electrode compartment (210), and
∘ a third interconnect layer (300) in the separation layer (230), electrically connecting to the electrodes (290) of the third set via a proximal end (291) thereof.

13. The microfluidic device (1) according to any one of claims 8 to 12, wherein at least one electrode among the first, second, and third set is hollow and comprises a conductive outer shell (130) forming the outer surface of the electrode, wherein the interconnect structure is hollow and has a conductive outer shell (141), and wherein a proximal end of said hollow electrode is fluidically connected to the hollow interconnect structure.

14. The microfluidic device (1) according to any one of claims 8 to 13, wherein the microfluidic device (1) comprises the three-dimensional biological cell structure (250) docked by the one or more electrodes (30) of the first set.

15. A system for culturing and monitoring three-dimensional biological cell structures (250), comprising:
i. the microfluidic device (1) according to any one of claims 7 to 14;
ii. a fluid control unit (310) configured to control fluid flow through the microfluidic compartment (200) and the through-hole (80), and
iii. an electronic measurement unit (320) configured to receive and process electrical signals from the first set of one or more electrodes (30).
